# FASCICULE DE BREVET EUROPEEN

(11) **EP 2 393 725 B1**
(45) Date de publication et mention de la délivrance du brevet: **04.03.2015**
(21) Numéro de dépôt: 10708261.2
(22) Date de dépôt: 01.02.2010
(51) Int. Cl.: B65D 47/18, A61F 9/00

(54) **EMBOUT AUTONETTOYANT**
SELBSTREINIGENDE SPITZE
SELF-CLEANING TIP

(30) Priorité: 03.02.2009 FR 0900433
(43) Date de publication de la demande: 14.12.2011
(73) Titulaire: Sivel, 06600 Antibes (FR)
(72) Inventeur: POZZI, Jacques, 06600 Antibes (FR); ROY, Pierre, 75019 Paris (FR)
(74) Mandataire: Cabinet Plasseraud
(86) Numéro de dépôt international: PCT/FR2010/050161
(87) Numéro de publication internationale: WO 2010/089501

(56) Documents cités:
- WO-A-00/13476
- WO-A1-2007/111256
- FR-A1- 2 873 358

## Description

La présente invention se rapporte au domaine technique de l'emballage, et plus spécialement à celui du conditionnement et de la distribution d'un produit liquide ou visqueux destiné à être conservé dans des conditions propres ou stériles, sans l'adjonction de conservateurs, et distribué par portions ou en doses, notamment en gouttes.

L'invention a plus particulièrement pour objet un dispositif de conditionnement et distribution d'un produit, et qui comprend un récipient destiné à contenir le produit devant être distribué à l'aide d'un accessoire de sortie dont le récipient est muni, ledit accessoire comprenant un embout.

On connaît des dispositifs de conditionnement de structure classique qui permettent de conserver et distribuer un produit sous forme de doses ou de gouttes calibrées ou sous toute autre forme, tout en maintenant sa propreté ou sa stérilité pendant toute la durée de son utilisation.

Ces dispositifs sont utilisés notamment dans les domaines pharmaceutique, cosmétique, et de l'alimentation, et pour certains plus particulièrement dans le domaine ophtalmologique.

Ainsi on connaît de tels dispositifs décrits dans les brevets FR 2 770 495, FR 2 638 428 et FR 2 661 401 dans lesquels un récipient est muni d'un embout distributeur dans lequel une membrane filtrante bactériologique est placée et permet de stériliser le produit lorsqu'il est expulsé du récipient.

Dans ces dispositifs, le récipient comporte une partie souple qui, lorsqu'elle est manuellement comprimée, provoque le passage du produit au travers de la membrane bactériologique et sa sortie à l'extérieur dudit récipient, au travers de l'embout.

Un autre type de dispositif qui permet d'obtenir un résultat analogue est décrit dans le brevet EP 08 61 128 dans lequel le produit est expulsé par le moyen d'une pompe munie d'un embout, ladite pompe comportant un filtre bactériologique placé dans le circuit de renouvellement d'air.

Un autre type de dispositif permettant d'obtenir un résultat analogue est décrit dans les brevets FR 2 873 358 et FR 2 892 397 dans lesquels le produit est expulsé au travers d'un embout souple formant accessoire, dont le récipient est muni, et qui s'ouvre sous l'effet de la pression créée par une pression sur les parois du récipient contenant le produit, ou sur l'embout lui-même, et qui se referme sous l'effet de son élasticité. Dans ce dispositif un filtre bactériologique est situé dans le fond du récipient qui est muni d'un orifice permettant le renouvellement de l'air.

D'autres dispositifs permettant d'obtenir un résultat similaire comportent une pompe distributrice du produit, munie d'un embout, et associée à un récipient sur lequel elle est assemblée, ledit récipient étant étanche et ne comportant pas de circuit de renouvellement de l'air.

L'ensemble de ces dispositifs qui comportent un filtre bactériologique permettant soit la filtration stérile du produit lui-même, soit la filtration stérile de l'air de renouvellement, ou qui ne comportent pas de filtre ni de circuit de renouvellement d'air dans le récipient, permettent la conservation propre ou stérile du produit à l'intérieur du récipient pendant la durée de son utilisation et peuvent être considérés, de ce point de vue, comme efficaces.

Ces dispositifs présentent par contre l'inconvénient majeur de ne pas assurer une propreté bactériologique parfaite à l'extérieur du récipient et plus particulièrement à l'extrémité de l'embout.

En effet, lorsqu'une goutte calibrée de produit a été expulsée par l'un des moyens que comportent ces dispositifs, conformément à leur destination, une fraction de cette goutte calibrée, que l'on dénommera goutte résiduelle, subsiste à l'extrémité de l'embout.

L'expérience a montré que, quel que soit le dispositif et le moyen mis en oeuvre pour expulser une goutte calibrée de produit, il n'est pas possible d'éviter la formation d'une goutte résiduelle qui subsistera à l'extrémité de l'embout jusqu'à la prochaine utilisation du dispositif.

La formation puis la présence de cette goutte résiduelle présente un inconvénient majeur puisque, contrairement au produit qui se trouve à l'intérieur du récipient et qui peut être maintenu propre ou stérile, cette goutte résiduelle reste au contact de l'air extérieur qui est susceptible de la contaminer.

Le but recherché par ces dispositifs existants et qui est de maintenir propre ou stérile, sans l'aide de conservateurs, le produit qu'ils renferment et distribuent, n'est pas atteint puisque, si le produit est effectivement maintenu propre ou stérile à l'intérieur du récipient, il peut être contaminé à l'extrémité de l'embout distributeur.

Différentes solutions ont été proposées pour résoudre ce problème qui affecte tous les dispositifs existants.

Ainsi, dans le dispositif décrit dans le brevet déjà cité FR 2 770 495, il est prévu de réaspirer la goutte résiduelle à l'intérieur du récipient, sous l'effet de la dépression formée par l'expulsion de la goutte calibrée, et de stériliser la goutte ré-aspirée par le moyen du filtre stérilisant placé dans le circuit de distribution du produit.

Cette solution, visant à éliminer la goutte résiduelle, n'est pas parfaite car la dépression créée à l'intérieur du récipient après l'expulsion de la goutte calibrée, n'est pas toujours suffisante pour vaincre la résistance du filtre stérilisant au passage de cette goutte résiduelle qui, de ce fait, peut ne pas être réaspirée vers l'intérieur du récipient.

Une autre solution a été proposée dans les dispositifs tels que ceux décrits dans le brevet EP 08 61 128, qui consiste à prévoir un insert métallique possédant des propriétés bactéricides, comme l'argent, et placé à l'intérieur de l'embout de manière à tuer les bactéries qui ont pu contaminer la goutte résiduelle.

Une solution voisine consiste à incorporer des matériaux bactéricides dans la matière plastique qui se trouve au contact de la goutte résiduelle.

De tels dispositifs prévoient également de laisser sécher la goutte résiduelle de manière à diminuer le risque de contamination bactérienne.

Ces solutions ne sont pas satisfaisantes, d'une part, parce que leur efficacité n'est que relative, et, d'autre part, parce qu'elles présentent le risque de relargage de molécules métalliques dans le produit, alors même que ces dispositifs ont été conçus pour éviter l'utilisation de conservateurs.

L'invention a pour but de permettre l'utilisation de dispositifs permettant de distribuer des gouttes calibrées de produit propre ou stérile, sans avoir l'inconvénient d'un risque de contamination bactérienne à l'extérieur dudit dispositif.

Plus généralement l'invention a pour but de remédier aux inconvénients des dispositifs analogues de l'état de la technique et de proposer un tel dispositif qui convienne mieux aux diverses exigences de la pratique que les dispositifs connus.

A l'effet d'atteindre le but recherché par l'invention, celle-ci propose un dispositif de conditionnement et distribution d'un produit selon le préambule de la revendication 1 et tel que connu par FR 2 892 397, et qui se caractérise par les mesures techniques de la partie caractérisante de la revendication 1. Des modes de réalisation avantageux font l'objet des revendications 2 à 8.

Ainsi le dispositif permet de distribuer des doses ou gouttes calibrées de produit propre ou stérile par simples pressions sur le récipient, sur l'accessoire ou sur l'embout dont l'accessoire est muni, tout en ayant l'assurance qu'aucune goutte résiduelle, susceptible d'être contaminée, ne subsistera à l'extrémité de l'embout après l'expulsion d'une dose ou goutte calibrée.

A titre d'exemple, et tel que décrit dans une variante du brevet FR 2 873 358, le dispositif qui permet de stocker un produit et le distribuer sous forme de gouttes calibrées stériles comporte un récipient muni d'un orifice pour l'arrivée de l'air de renouvellement et un filtre permettant de stériliser ledit air lorsqu'il pénètre à l'intérieur dudit récipient. Le produit est stocké à l'intérieur dudit récipient et distribué à l'aide d'un embout formant accessoire dont ledit récipient est muni. Cet embout comporte une chambre de dosage permettant de déterminer le volume de la goutte calibrée et qui, lorsqu'elle est comprimée, par exemple, à l'aide d'un bouton poussoir, permet à ladite goutte d'être expulsée à l'extérieur dudit récipient au moyen de l'ouverture d'un clapet anti-retour empêchant la pénétration d'air de l'extérieur vers la chambre de dosage.

WO 00/13476A décrit un dispositif de distribution d'un produit sous forme de gouttes, qui comprend des moyens permettant soit d'aspirer une goutte résiduelle, soit de souffler ladite goutte résiduelle, soit de souffler la goutte sans permettre la formation d'une goutte résiduelle.

La chambre complémentaire peut être située à l'extérieur de l'embout comme à l'intérieur de l'embout.

Avantageusement, et dans une première forme de réalisation de la présente invention, une pièce complémentaire est placée à l'extérieur de l'embout dans lequel se trouve cette chambre de dosage. Cette pièce complémentaire est réalisée dans un matériau de préférence souple et élastique et elle est fixée solidairement et d'une manière étanche à la base dudit embout qu'elle entoure. Cette pièce complémentaire est réalisée et assemblée à l'embout de telle façon qu'un volume libre est crée entre cette pièce complémentaire et l'embout lui-même. Ce volume libre constitue la chambre complémentaire qui est comprimée puis relâchée automatiquement lorsque la chambre de dosage est elle même comprimée puis relâchée, et qui sert soit à aspirer la goutte résiduelle, soit à la souffler, soit à souffler la goutte calibrée elle même tout en empêchant la création d'une goutte résiduelle.

Dans une première variante, l'extrémité de la pièce complémentaire comporte un orifice de sortie de forme cylindrique et est assemblée d'une manière étanche avec l'extrémité de l'embout du côté de la chambre de dosage et d'une manière non étanche du côté opposé à la chambre de dosage. Ainsi, lorsqu'une goutte calibrée a été expulsée après compression de la chambre de dosage et de la chambre complémentaire, la goutte résiduelle qui s'est créée et qui subsiste dans l'orifice de sortie cylindrique de la pièce complémentaire est aspirée à l'intérieur de la chambre complémentaire lorsque l'ensemble constitué par la chambre de dosage et la chambre complémentaire est relâché.

Dans une autre forme de réalisation, la partie de la pièce extérieure située du côté de la chambre de dosage peut ne pas être directement au contact d'un bouton poussoir de la chambre de dosage. En d'autres termes, la paroi de la chambre complémentaire peut ne pas être en contact d'un poussoir de la chambre de dosage. Ainsi, lorsque le dispositif est utilisé, l'air contenu dans la chambre complémentaire est expulsé avant que la goutte calibrée ne soit elle-même expulsée. Cet effet retard permet au dispositif de ne pas expulser d'air en même temps que la goutte calibrée. D'autres moyens de créer un effet retard peuvent être mis en oeuvre comme, par exemple, une différence d'épaisseur entre les parois de la chambre complémentaire, la paroi située à l'opposé de la chambre de dosage étant moins épaisse donc plus souple que celle située du côté de la chambre de dosage.

Ainsi, la paroi de la chambre complémentaire peut être moins épaisse que la paroi de la chambre de dosage, de manière à permettre l'expulsion de l'air contenu dans la chambre complémentaire avant l'expulsion de la goutte calibrée de la chambre de dosage.

Dans une autre variante, l'extrémité cylindrique de la chambre complémentaire est assemblée avec l'extrémité de l'embout de manière étanche du côté de la chambre de dosage ainsi que du côté opposé à cette chambre de dosage. Ainsi, la goutte résiduelle peut être soufflée vers l'extérieur de l'embout sans pouvoir être aspirée à l'intérieur de la chambre complémentaire. Dans ce cas il est indispensable que la chambre de dosage expulse la goutte calibrée avant que la chambre complémentaire n'expulse l'air qui sert à souffler la goutte résiduelle. A cet effet différents moyens peuvent être mis en oeuvre afin que la compression de la chambre complémentaire soit retardée par rapport à celle de la chambre de dosage. Ainsi, la paroi de la pièce complémentaire située du côté opposé à la chambre de dosage peut être plus épaisse, et donc plus rigide, que celle située du côté de la chambre de dosage. En d'autres termes, la paroi de la chambre de dosage peut être moins épaisse que la paroi de la chambre complémentaire de manière à permettre l'expulsion de la goutte calibrée de la chambre de dosage avant l'expulsion de l'air contenu dans la chambre complémentaire. De même, on peut envisager la création d'un orifice situé dans la chambre complémentaire et coopérant avec une butée située sur une partie de l'embout, de telle manière que cet orifice reste ouvert et s'oppose à toute compression de l'air contenu dans la chambre complémentaire pendant que la chambre de dosage expulse la goutte calibrée, puis soit obturé après que la goutte calibrée ait été expulsée et permette ainsi l'expulsion de l'air contenu dans la chambre complémentaire, par compression de cette chambre et ainsi le soufflage de la goutte résiduelle. Ainsi, la chambre complémentaire peut comprendre un orifice destiné à permettre l'expulsion de la goutte calibrée de la chambre de dosage avant l'expulsion de l'air contenu dans la chambre complémentaire.

Afin que la chambre complémentaire puisse se remplir à nouveau d'air après que la goutte résiduelle a été soufflée par l'air contenu précédemment dans ladite chambre complémentaire, on peut prévoir un second orifice dans la paroi de ladite chambre complémentaire. Ce second orifice est obturé automatiquement par la pression du doigt de l'utilisateur lorsqu'il appuie simultanément du côté de la chambre de dosage et du côté de la chambre complémentaire, afin de faire fonctionner le dispositif, et s'ouvre automatiquement lorsque l'utilisateur cesse d'appuyer sur les deux faces de l'embout, permettant ainsi à l'air de pénétrer à nouveau à l'intérieur de la chambre complémentaire. Ainsi, un poussoir ou la paroi de la chambre complémentaire peut comporter au moins un orifice destiné à permettre l'entrée de l'air de remplacement dans ladite chambre complémentaire.

Dans une autre forme de réalisation, la chambre complémentaire, permettant soit l'aspiration, soit le soufflage de la goutte résiduelle, n'est pas obtenue à l'aide d'une pièce complémentaire entourant l'embout, mais est située à l'intérieur de l'embout lui-même.

Cette forme de réalisation permet d'obtenir le même résultat que celui obtenu par la création d'une chambre complémentaire située à l'extérieur de l'embout, mais elle comporte l'avantage d'éviter l'ajout d'une pièce supplémentaire.

Ainsi cette chambre complémentaire est réalisée en opposition avec la chambre de dosage de manière à être comprimée et relâchée automatiquement lorsque ladite chambre de dosage est comprimée par appui sur les deux faces de l'embout, dans le but d'obtenir la délivrance d'une goutte calibrée, puis relâchée après la délivrance de cette goutte.

Des moyens sont mis en oeuvre de manière à assurer une étanchéité parfaite entre les deux chambres afin d'éviter toute communication d'air ou de produit entre elles.

Les moyens imaginés et décrits précédemment pour le fonctionnement de la chambre complémentaire extérieure à l'embout, afin de permettre soit l'aspiration de la goutte résiduelle soit son soufflage, sont identiques, dans le cas d'une chambre complémentaire intérieure.

Ainsi, dans le cas de l'aspiration de la goutte résiduelle, un passage est ménagé à l'extrémité de l'embout afin de permettre l'expulsion de l'air contenu dans la chambre complémentaire puis l'aspiration de la goutte résiduelle vers l'intérieur de ladite chambre complémentaire. De même, les épaisseurs des parois des deux chambres sont différentes, ce qui permet d'obtenir un effet retard et l'expulsion de l'air contenu dans la chambre complémentaire préalablement à l'expulsion de la goutte calibrée.

Dans le cas du soufflage de la goutte résiduelle la suppression de ce passage permet d'éviter que du produit ne puisse être aspiré vers l'intérieur de la chambre complémentaire, et des différences d'épaisseur entre les parois des deux chambres permettent d'obtenir un effet retard de manière que la goutte calibrée puisse être distribuée avant que l'air contenu dans la chambre complémentaire n'expulse la goutte résiduelle.

De la même manière, il peut être prévu, dans la chambre complémentaire, un orifice qui ne s'obture qu'après une certaine compression de ladite chambre de manière à laisser à la goutte calibrée un temps suffisant afin qu'elle soit expulsée de la chambre de dosage avant que l'air utilisé pour le soufflage de la goutte résiduelle ne soit lui-même expulsé de la chambre complémentaire.

Un second orifice, permettant à la chambre complémentaire de se remplir d'air après le soufflage de la goutte résiduelle, peut également être prévu dans cette variante, où ladite chambre est située à l'intérieur de l'embout.

Ainsi, la création d'une chambre complémentaire à la chambre de dosage, qu'elle soit située à l'extérieur ou à l'intérieur de l'embout, associée à des moyens lui permettant soit d'aspirer la goutte résiduelle, soit de la souffler sans interférer avec la distribution de la goutte calibrée, permet d'une manière avantageusement simple d'éviter que ladite goutte résiduelle subsiste à l'extrémité de l'embout et soit susceptible d'être polluée.

La loi de Tate nous enseigne que la chute d'une goutte calibrée dépend de sa masse et du diamètre de l'orifice à l'extrémité duquel elle se forme. Pour un diamètre d'orifice donné la goutte calibrée chute d'elle même à partir d'une masse que l'on qualifie de masse critique. Dans le dispositif objet de l'invention, et dans le cas de la variante précédemment décrite permettant de souffler la goutte résiduelle, il peut être avantageux de prévoir une chambre de dosage de dimensions telles que la goutte calibrée ait une masse inférieure à cette masse critique, et donc qu'elle ne chute pas d'elle même. Ainsi, c'est la goutte calibrée elle-même qui sera soufflée sans qu'il n'y ait formation d'une goutte résiduelle.

On réalise ainsi, d'une manière simple, un dispositif permettant de distribuer des gouttes calibrées stériles, sans qu'aucune goutte résiduelle susceptible d'être polluée par l'environnement extérieur, ne subsiste à l'extrémité de l'embout.

D'autres caractéristiques et avantages de l'invention ressortent de la description donnée ci-dessous, en référence aux dessins annexés, qui représentent, à titre d'exemples non limitatifs, des formes de réalisation et de mise en oeuvre de l'objet de l'invention. Sur ces dessins :
- la figure 1 est une vue schématique en coupe axiale d'un dispositif représentant un récipient muni d'un dispositif de renouvellement de l'air au travers d'un filtre stérile et d'un embout formant accessoire comportant une chambre de dosage et deux clapets permettant la distribution de gouttes calibrées;
- la figure 2 est une vue en coupe axiale du dispositif représenté dans la figure 1 après sollicitation et expulsion d'une goutte calibrée et formation d'une goutte résiduelle;
- la figure 3 est une vue schématique en coupe axiale du dispositif conforme à l'invention sur laquelle est représenté un embout, faisant partie de l'ensemble représenté dans la figure 1, dans laquelle apparaît une chambre complémentaire située à l'intérieur de l'embout lui même, ainsi qu'un canal ouvert à son extrémité;

- la figure 4 est une vue analogue à la figure 3 lorsque le dispositif est sollicité et que seule la chambre complémentaire a été comprimée;
- la figure 5 est une vue analogue à la figure 4 lorsque le dispositif est sollicité et que la chambre de dosage est comprimée;
- la figure 6 est une vue analogue à la figure 5 lorsque le dispositif n'est plus sollicité;
- la figure 7 est une vue analogue à la figure 3 et dans laquelle apparaît une chambre complémentaire munie de deux orifices et d'un canal fermé à son extrémité;
- la figure 7a est une vue agrandie de la partie de la figure 7 sur laquelle apparaissent les deux orifices;
- la figure 8 est une vue analogue à la figure 7 lorsque le dispositif est sollicité et que seule la chambre de dosage est comprimée;
- la figure 9 est une vue analogue à la figure 8 lorsque le dispositif est sollicité et que la chambre complémentaire est comprimée;
- la figure 10 est une vue schématique en coupe axiale du dispositif conforme à l'invention et sur laquelle est représenté un embout, faisant partie de l'ensemble représenté dans la figure 1, entouré d'une pièce souple et déformable; et
- la figure 11 est une vue analogue à la figure 10, et dans laquelle apparaissent deux orifices dans la pièce souple et déformable entourant l'embout, ainsi que des différences d'épaisseur dans les parois constituant cette pièce souple et déformable.

Sur les différentes figures, les mêmes références numériques désignent des éléments analogues des différents exemples de réalisation représentés et décrits.

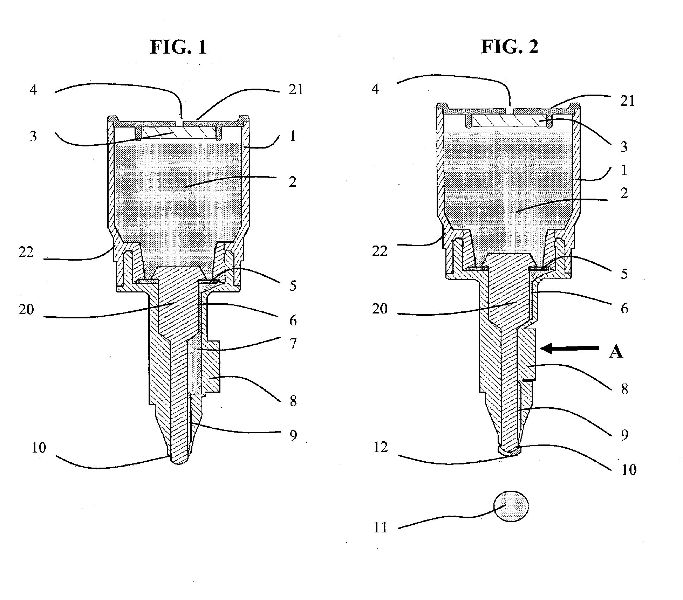

Tel que représenté sur la figure 1, le dispositif de conditionnement et distribution comprend un récipient 1, composé d'un fond 21 dans lequel se trouve un ensemble de renouvellement et filtration d'air comprenant un passage d'entrée d'air 4 débouchant sur un filtre à air 3, et d'une partie d'extrémité supérieure 22 solidaire d'un embout 20, dans lequel se trouvent une chambre de dosage 7, actionnée par un poussoir 8 et entourée de deux clapets 5 et 10 ainsi que de deux canaux d'alimentation 6 et 9 pour la distribution du produit 2 contenu dans le corps du récipient 1.

Le premier clapet 10 permet le passage vers l'extérieur du produit 2 contenu dans la chambre de dosage 7, par l'intermédiaire du canal 9, lorsque ladite chambre est comprimée par appui sur le poussoir 8, sans que l'air extérieur puisse entrer à l'intérieur de ladite chambre de dosage 7 lorsque ledit poussoir 8 est relâché; le second clapet 5 permet l'admission du produit 2, contenu dans le récipient 1, dans la chambre de dosage 7 par l'intermédiaire du canal 6 lorsque le poussoir est relâché sans que le produit 2 contenu dans la chambre de dosage 7 puisse retourner dans le récipient 1 lorsque ladite chambre est comprimée.

La figure 2 représente ce même dispositif lorsqu'il a été sollicité par appui sur le poussoir 8 suivant la flèche A, provoquant la compression de la chambre de dosage 7, l'expulsion d'une goutte calibrée 11 au travers du clapet 10 et la formation d'une goutte résiduelle 12.

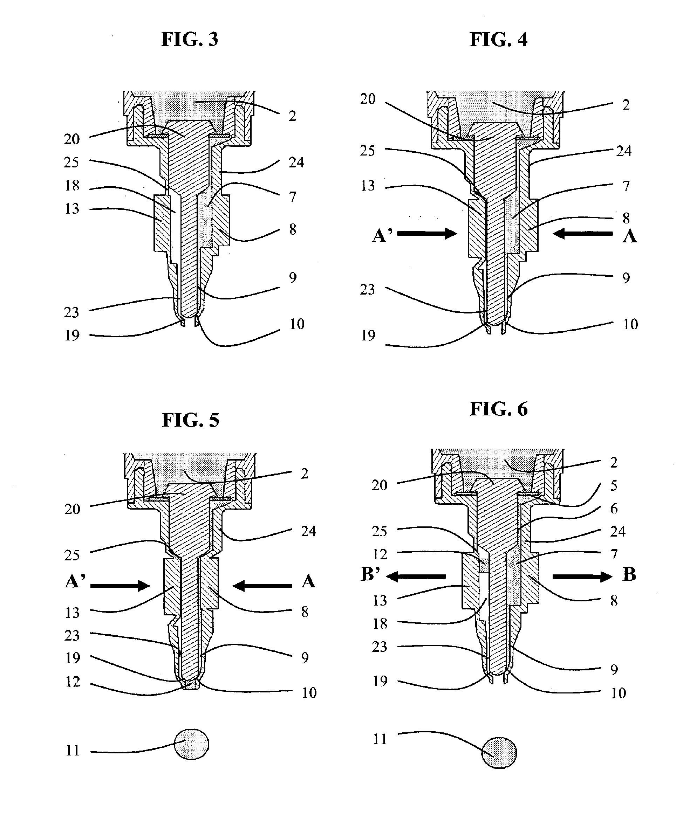

La figure 3 représente l'embout 20 à l'intérieur duquel a été aménagée une chambre complémentaire 18, située à l'opposé de la chambre de dosage 7 dont elle est séparée par des moyens étanches empêchant toute communication entre elles, et actionnée par un poussoir 13, ainsi qu'un canal 23 ouvert à son extrémité 19. La paroi 24 de la chambre de dosage 7 est plus épaisse que la paroi 25 de la chambre complémentaire 18 de sorte que, lorsque le dispositif est sollicité par appui simultané sur les poussoirs 8 et 13, la chambre complémentaire est comprimée avant la chambre de dosage.

Le dispositif fonctionne de la façon suivante en référence aux figures 4, 5 et 6.

Une pression manuelle simultanément exercée par un utilisateur sur les poussoirs 8 et 13, suivant les flèches A et A', entraînera dans un premier temps (figure 4) la compression de la chambre complémentaire 18, la paroi 25 de cette chambre complémentaire 18 étant moins épaisse, donc moins résistante à l'effort, que la paroi 24 de la chambre de dosage 7 qui, elle, n'est pas encore comprimée.

Cette compression anticipée de la chambre complémentaire 18, avant celle de la chambre de dosage 7, a pour effet d'expulser l'air contenu dans cette chambre complémentaire 18, par l'intermédiaire du canal 23 et de son extrémité 19, avant l'expulsion d'une goutte calibrée 11 de produit 2, de manière à ne pas expulser un mélange d'air et de produit.

Dans un second temps (figure 5) la pression simultanée sur les poussoirs 8 et 13 entraînera la compression de la chambre de dosage 7 et l'expulsion d'une goutte calibrée 11 de produit 2 par l'intermédiaire du canal 9 et l'ouverture du clapet 10 ainsi que la formation d'une goutte résiduelle 12.

Dans un troisième temps (figure 6) le relâchement de la pression par l'utilisateur, suivant les flèches B et B', et la reprise des formes initiales des chambres 7 et 18 a pour conséquence d'une part le remplissage de la chambre de dosage 7 par du produit 2 par ouverture du clapet 5 et par l'intermédiaire du canal 6, sans que de l'air extérieur ne puisse pénétrer dans ladite chambre de dosage, le clapet 10 étant élastiquement fermé, et d'autre part l'aspiration par la chambre complémentaire 18 de la goutte résiduelle 12 par l'intermédiaire de l'orifice 19 et du canal 23.

La communication entre la chambre complémentaire 18 et l'air extérieur, par l'intermédiaire du canal 23 et de l'orifice 19, a pour conséquence le séchage de la goutte résiduelle préalablement aspirée dans ladite chambre 18.

On obtient ainsi par aspiration puis séchage la disparition complète de la goutte résiduelle de l'extrémité de l'embout.

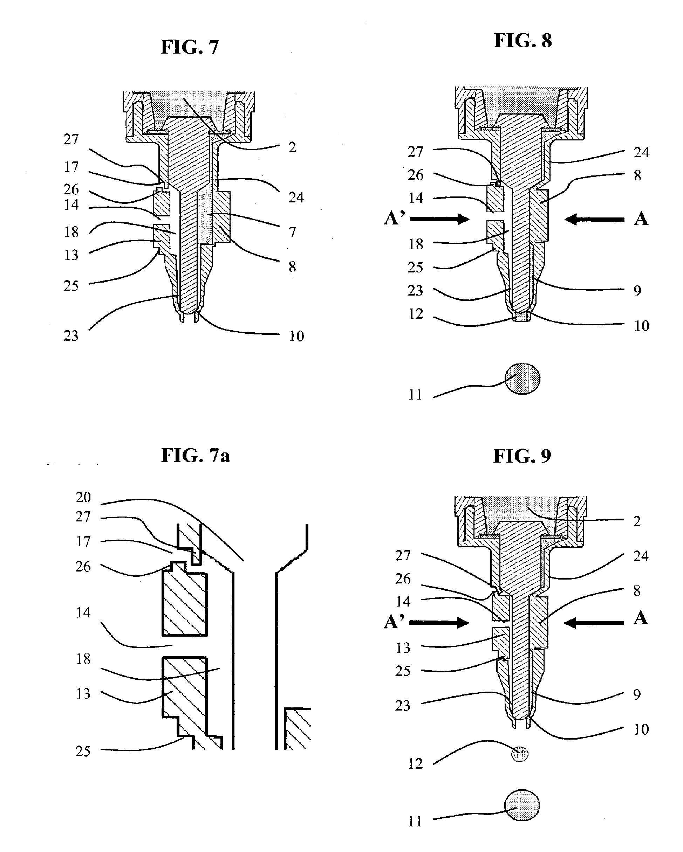

La figure 7 représente une variante du dispositif dans lequel l'extrémité du canal 23 de la chambre complémentaire 18 est fermée et la paroi 25 de la chambre complémentaire 18 est plus épaisse que la paroi 24 de la chambre de dosage 7.

Ainsi une pression manuelle simultanée d'un utilisateur sur les poussoirs 8 et 13 suivant les flèches A et A' (figure 8) a pour effet de comprimer d'abord la chambre de dosage 7 qui possède la paroi la plus fine donc la moins résistante à l'effort, provoquant l'expulsion d'une goutte calibrée 11 de produit 2 par l'intermédiaire du canal 9 et du clapet 10, ainsi que la formation d'une goutte résiduelle 12, avant la compression de la chambre complémentaire 18 qui possède la paroi 25 la plus épaisse, donc la plus résistante à l'effort, et l'expulsion de l'air qu'elle renferme.

L'expulsion de la goutte calibrée 11 préalablement à celle de l'air contenu dans la chambre complémentaire 18 est nécessaire afin d'éviter l'expulsion d'un mélange d'air et de produit 2. Cette expulsion anticipée de la goutte calibrée 11 de produit 2 peut être obtenue par d'autres moyens que des différences d'épaisseurs entre les parois des deux chambres.

Ainsi les figures 7 et 7a font apparaître un orifice 17, situé dans la paroi 25 de la chambre complémentaire 18, ainsi qu'une lèvre 26 coopérant avec une lèvre 27. Cet orifice 17 (figure 7a) est ouvert lorsque le dispositif n'est pas sollicité. Lorsqu'un utilisateur appuie simultanément sur les poussoirs 8 et 13, ce qui a pour effet de comprimer les deux chambres 7 et 18, l'air contenu dans la chambre complémentaire 18 s'échappe par l'orifice 17 jusqu'à ce que les deux lèvres 26 et 27 viennent au contact l'une de l'autre et obture ledit orifice 17. Pendant ce laps de temps la chambre 7 est comprimée et une goutte calibrée 11 de produit 2 est expulsée.

Par ce moyen supplémentaire on évite que de l'air provenant de la chambre complémentaire 18 soit expulsé en même temps que la goutte calibrée 11 de produit 2.

La figure 8 représente le dispositif dans une position telle qu'une action d'un utilisateur sur les poussoirs 8 et 13 suivant les flèches A et A' provoque l'expulsion d'une goutte calibrée 11 par compression de la chambre de dosage 7 et le déplacement du poussoir 13 sur une distance telle que l'orifice 17 commence à être obturé par le contact entre les deux lèvres 26 et 27 sans que la chambre complémentaire n'ait été encore comprimée.

La présence de l'orifice 17 et de son obturation par les lèvres coopérantes 26 et 27 avec retard, empêche toute compression de la chambre complémentaire 18 pendant que la goutte calibrée est expulsée et permet ainsi d'éviter l'expulsion d'un mélange d'air et de produit 2.

La figure 9 représente le dispositif lorsque l'utilisateur continue à le solliciter par appui sur les poussoirs 8 et 13 et que la chambre complémentaire 18, dont l'orifice d'échappement d'air 17 a été obturé, est comprimée, provoquant l'expulsion de l'air par le canal 23, l'ouverture du clapet 10 et le soufflage de la goutte résiduelle 12.

Si la masse de la goutte calibrée 11, déterminée par le volume de la chambre de dosage 7, est telle qu'elle soit inférieure à la masse critique suivant la loi de Tate, ladite goutte calibrée 11 ne tombe pas d'elle-même, elle reste accrochée à l'extrémité de l'embout 2 et est confondue avec la goutte résiduelle 12.

Ainsi lorsque la chambre complémentaire 18 est comprimée, après fermeture de l'orifice 17, c'est la goutte calibrée 11 elle-même qui est soufflée sans même qu'il y ait formation d'une goutte résiduelle 12.

Afin que la chambre complémentaire 18 puisse se remplir à nouveau d'air il est prévu un trou 14 ( figure 7 et 7a) qui est bouché par le doigt de l'utilisateur lorsque ce dernier exerce une pression sur le poussoir 13 suivant la flèche A' (figures 8 et 9) et ouvert lorsque l'utilisateur relâche sa pression.

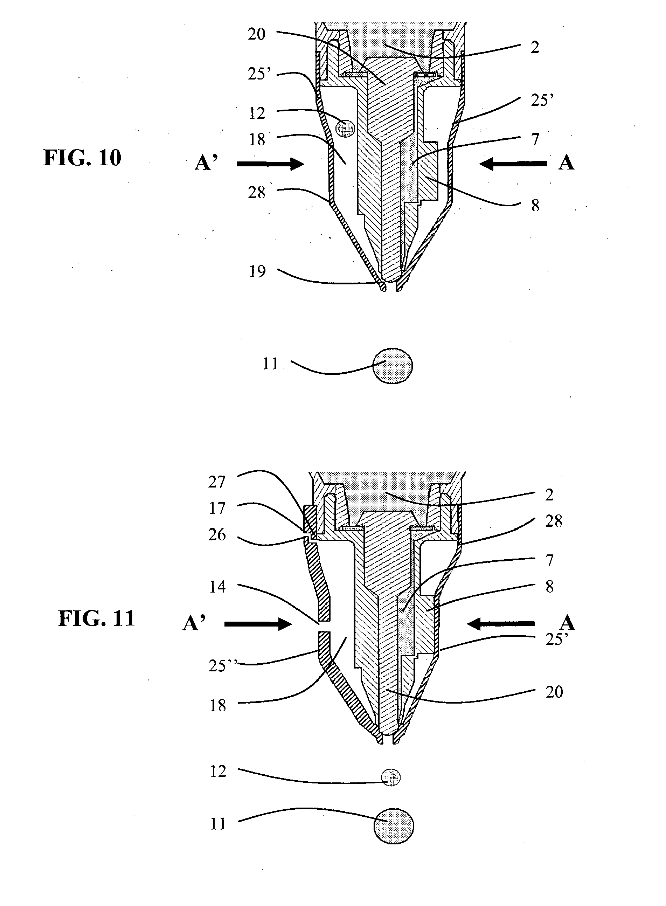

La figure 10 représente l'embout 20 entouré d'une pièce souple extérieure 28 formant une chambre complémentaire 18 opposée à la chambre de dosage 7. La paroi 25' de la pièce extérieure 28 n'est pas au contact du poussoir 8 et l'intérieur de ladite chambre complémentaire est en communication avec l'air extérieur par l'intermédiaire du canal 19.

Le dispositif représenté est conforme à l'invention et fonctionne d'une manière identique à celui représenté sur la figure 3 dans lequel la chambre complémentaire 18 est située à l'intérieur de l'embout.

Lorsque le dispositif est sollicité et qu'un utilisateur exerce une pression sur les parois de la pièce souple suivant les flèches A et A', le fait que la paroi 25' ne soit pas au contact du poussoir 8 permet, dans un premier temps, l'expulsion de l'air contenu dans la chambre complémentaire 18 avant que la chambre de dosage 7 soit elle-même comprimée par appui de la paroi 25' sur le poussoir 8 et qu'une goutte calibrée 11 soit expulsée. On a ainsi évité que de l'air soit expulsé en même temps que du produit 2.

Lorsque l'utilisateur relâche sa pression la chambre complémentaire reprend sa forme et aspire la goutte résiduelle 12 qui s'est formée après l'expulsion de la goutte calibrée 11 dans les mêmes conditions que le dispositif représenté sur la figure 6.

La figure 11 représente un dispositif comprenant un embout 20 entouré d'une pièce souple extérieure 28 dont la paroi 25' est au contact du poussoir 8 mais est plus fine que la paroi opposée 25" qui comprend elle-même deux orifices 17 et 14.

Le fonctionnement de ce dispositif est identique à celui représenté sur les figures 7, 8 et 9 et il permet de souffler soit la goutte résiduelle après l'expulsion d'une goutte calibrée, soit la goutte calibrée elle-même, sans qu'il n'y ait formation d'une goutte résiduelle, lorsque sa masse a été choisie de manière qu'elle ne tombe pas d'elle-même.

Le même effet retard de la compression de la chambre complémentaire 18 par rapport à celle de la chambre de dosage 7 est obtenu par des moyens identiques à ceux décrits sur les figures 7, 7a, 8 et 9 et le trou 14 permet à la chambre complémentaire 18 de se remplir d'air dans les mêmes conditions.

D'autres modes de réalisation de la chambre complémentaire associée à l'embout sont possibles sans sortir du cadre de l'invention dès lors que cette chambre a pour fonction d'aspirer la goutte résiduelle ou de la souffler ou de souffler la goutte calibrée elle-même sans permettre la formation de la goutte résiduelle.

D'autres moyens analogues peuvent également être mis en oeuvre afin d'empêcher toute expulsion simultanée d'air à partir de la chambre complémentaire et de produit à partir de la chambre de dosage sans sortir du cadre de l'invention, comme défini par les revendications 1-8.

## Revendications

1. Dispositif de conditionnement et distribution d'un produit, généralement liquide ou visqueux, comprenant un récipient (1) destiné à contenir le produit (2) à conditionner et distribuer sous forme de doses ou gouttes calibrées (11) propres ou stériles à l'aide d'un accessoire de distribution muni d'un embout (20), l'ensemble du récipient (1) et de l'accessoire comportant une chambre de dosage (7) de produit, alimentée en produit (2) à partir du récipient (1) par un premier clapet (5)anti-retour, et mise en communication avec l'extérieur du dispositif, pour délivrer une dose ou goutte calibrée (11) de produit, par un second clapet (10) anti-retour, lorsque ledit accessoire est actionné,
**caractérisé en ce qu'**il comprend de plus une chambre complémentaire (18) souple et déformable, actionnable simultanément avec la chambre de dosage (7), et permettant soit d'aspirer une goutte résiduelle (12) présente à l'extrémité dudit accessoire après la distribution d'une dose ou goutte calibrée (11), soit de souffler ladite goutte résiduelle (12), soit de souffler ladite dose ou goutte calibrée (11) sans permettre la formation d'une goutte résiduelle (12).

2. Dispositif selon la revendication 1 , **caractérisé en ce que** ladite chambre complémentaire (18) est située à l'intérieur de l'embout (20) lui-même.

3. Dispositif selon la revendication 1, **caractérisé en ce que** ladite chambre complémentaire (18) est située à l'extérieur de l'embout (20).

4. Dispositif selon la revendication 2 **caractérisé en ce que** la paroi (25) de la chambre complémentaire (18) est moins épaisse que la paroi (24) de la chambre de dosage (7) de manière à permettre l'expulsion de l'air contenu dans la chambre complémentaire (18) avant l'expulsion de la goutte calibrée (11) de la chambre de dosage (7).

5. Dispositif selon l'une des revendications 2 et 3, **caractérisé en ce que** la paroi (24) de la chambre de dosage (7) est moins épaisse que la paroi (25, 25") de la chambre complémentaire (18) de manière à permettre l'expulsion de la goutte calibrée (11) de la chambre de dosage (7) avant l'expulsion de l'air contenu dans la chambre complémentaire (18).

6. Dispositif selon l'une des revendications 2 et 3, **caractérisé en ce que** la chambre complémentaire (18) comprend un orifice (17) destiné à permettre l'expulsion de la goutte calibrée (11) de la chambre de dosage (7) avant l'expulsion de l'air contenu dans la chambre complémentaire (18).

7. Dispositif suivant l'une des revendications 2 et 3, **caractérisé en ce qu'**un poussoir (13) ou la paroi (25") de la chambre complémentaire (18) comporte au moins un orifice (14) destiné à permettre l'entrée de l'air de remplacement dans ladite chambre (18).

8. Dispositif suivant la revendication 3, **caractérisé en ce que** la paroi (25') de la chambre complémentaire (18) n'est pas au contact d'un poussoir (8) de la chambre de dosage (7).

## Patentansprüche

1. Vorrichtung zum Verpacken und Spenden eines im Allgemeinen flüssigen oder zähflüssigen Produkts, umfassend einen Behälter (1), der dazu gedacht ist, das Produkt (2) zu enthalten, das in Form von kalibrierten, sauberen oder sterilen Dosierungen oder Tropfen (11) anhand eines Spenderzubehörteils, das mit einem Endstück (20) versehen ist, zu verpacken und abzugeben ist, wobei die Einheit aus Behälter (1) und Zubehörteil eine Produktdosierungskammer (7) umfasst, die aus dem Behälter (1) über eine erste Rückschlagklappe (5) mit dem Produkt (2) versorgt wird, und mit dem Äußeren der Vorrichtung in Verbindung steht, um eine kalibrierte Dosierung oder einen kalibrierten Tropfen (11) des Produkts über eine zweite Rückschlagklappe (10) abzugeben, wenn das Zubehörteil betätigt wird,
**dadurch gekennzeichnet, dass** sie des Weiteren eine biegsame und verformbare Zusatzkammer (18) umfasst, die gleichzeitig mit der Dosierungskammer (7) betätigt werden kann und es ermöglicht, entweder einen Resttropfen (12) anzusaugen, der nach dem Spenden einer kalibrierten Dosierung oder eines kalibrierten Tropfens (11) am Ende des Zubehörteils vorliegt, oder den Resttropfen (12) wegzublasen oder die kalibrierte Dosierung oder den kalibrierten Tropfen (11) wegzublasen, ohne die Bildung eines Resttropfens (12) zu erlauben.

2. Vorrichtung nach Anspruch 1, **dadurch gekennzeichnet, dass** sich die Zusatzkammer (18) innerhalb des Endstücks (20) selber befindet.

3. Vorrichtung nach Anspruch 1, **dadurch gekennzeichnet, dass** sich die Zusatzkammer (18) außerhalb des Endstücks (20) befindet.

4. Vorrichtung nach Anspruch 2, **dadurch gekennzeichnet, dass** die Wand (25) der Zusatzkammer (18) weniger dick ist als die Wand (24) der Dosierungskammer (7), um das Ausstoßen der Luft, die in der Zusatzkammer (18) enthalten ist, vor dem Ausstoßen des kalibrierten Tropfens (11) aus der Dosierungskammer (7) zu ermöglichen.

5. Vorrichtung nach einem der Ansprüche 2 und 3, **dadurch gekennzeichnet, dass** die Wand (24) der Dosierungskammer (7) weniger dick ist als die Wand (25, 25") der Zusatzkammer (18), um das Ausstoßen des kalibrierten Tropfens (11) aus der Dosierungskammer (7) vor dem Ausstoßen der Luft, die in der Zusatzkammer (18) enthalten ist, zu ermöglichen.

6. Vorrichtung nach einem der Ansprüche 2 und 3, **dadurch gekennzeichnet, dass** die Zusatzkammer (18) eine Öffnung (17) umfasst, die dazu gedacht ist, das Ausstoßen des kalibrierten Tropfens (11) aus der Dosierungskammer (7) vor dem Ausstoßen der Luft, die in der Zusatzkammer (18) enthalten ist, zu ermöglichen.

7. Vorrichtung nach einem der Ansprüche 2 und 3, **dadurch gekennzeichnet, dass** ein Stößel (13) oder die Wand (25") der Zusatzkammer (18) mindestens eine Öffnung (14) umfasst, die dazu gedacht ist, den Eintritt der Ersatzluft in die Kammer (18) zu ermöglichen.

8. Vorrichtung nach Anspruch 3, **dadurch gekennzeichnet, dass** die Wand (25') der Zusatzkammer (18) nicht mit einem Stößel (8) der Dosierungskammer (7) in Kontakt steht.

## Claims

1. A device for packaging and dispensing a generally liquid or viscous product, comprising a container (1) for containing the product (2) to be packaged and dispensed in the form of clean or sterile measured doses or drops (11) by means of a dispensing accessory including a tip (20), wherein the assembly of container (1) and accessory comprises a product dosing chamber (7), supplied with product (2) from the container (1) by a first non-return valve (5), and in communication with outside the device by means of a second non-return valve (10) in order to dispense a measured dose or drop (11) of product, when said accessory is actuated,
**characterized in that** it additionally comprises a flexible and deformable supplemental chamber (18), actuatable simultaneously with said closing chamber (7) and enabling to either suction a residual drop (12) present at the end of said accessory after the dispensing of a measured dose or drop (11), or blow said residual drop (12), or blow said measured dose or drop (11) without permitting the formation of a residual drop (12).

2. A device according to claim 1, **characterized in that** said supplemental chamber (18) is situated inside the tip (20) itself.

3. A device according to claim 1, **characterized in that**, said supplemental chamber (18) is situated outside the tip (20).

4. A device according to claim 2 **characterized in that** the wall (25) of the supplemental chamber (18) is thinner than the wall (24) of the dosing chamber (7) such that the air contained in the supplemental chamber (18) is expelled before the measured drop (11) is expelled from the dosing chamber (7).

5. A device according to either of claims 2 or 3, **characterized in that** the wall (24) of the dosing chamber (7) is thinner than the wall (25, 25") of the supplemental chamber (18) such that the measured drop (11) is expelled from the dosing chamber (7) before the air contained in the supplemental chamber (18) is expelled.

6. A device according to either of claims 2 or 3 **characterized in that** the supplemental chamber (18) comprises an opening (17) such that the measured drop (11) is expelled from the dosing chamber (7) before the air contained in the supplemental chamber (18) is expelled.

7. A device according to either of claims 2 or 3, **characterized in that** a pushbutton (13) or the wall (25") of the supplemental chamber (18) comprises at least one opening (14) to allow the intake of replacement air into said chamber (18).

8. A device according to claim 3, **characterized in that** the wall (25') of the supplemental chamber (18) is not in contact with a pushbutton (8) of the dosing chamber (7).
